# NEUE EUROPÄISCHE PATENTSCHRIFT

(11) **EP 2 411 354 B2**
(45) Veröffentlichungstag und Bekanntmachung des Hinweises auf die Entscheidung über den Einspruch: **04.10.2017**
(45) Hinweis auf die Patenterteilung: 27.11.2013
(21) Anmeldenummer: 10710284.0
(22) Anmeldetag: 18.03.2010
(51) Int. Cl.: C08G 63/16, C08K 5/13, C08K 5/375

(54) **STABILISIERUNG VON POLYOLEN**
STABILIZATION OF POLYOLS
STABILISATION DE POLYOLS

(30) Priorität: 26.03.2009 DE 102009014411
(43) Veröffentlichungstag der Anmeldung: 01.02.2012
(73) Patentinhaber: LANXESS Deutschland GmbH, 50569 Köln (DE)
(72) Erfinder: HEUER, Lutz, 41542 Dormagen (DE)
(86) Internationale Anmeldenummer: PCT/EP2010/053507
(87) Internationale Veröffentlichungsnummer: WO 2010/108840

(56) Entgegenhaltungen:
- EP-A1- 0 556 612
- EP-A2- 0 572 256
- EP-A2- 0 849 296
- WO-A1-2006//092363
- WO-A1-2007//122124
- DE-A1-102004 002 094
- GB-A- 2 015 505
- JP-A- 10 -25 324
- JP-A- 06- 128 364
- US-A- 4 070 304
- US-A- 4 162 363
- DATABASE WPI Thomson Scientific, London, GB; AN 1978-77545A XP002578514 -& JP 53 108909 A 22. September 1978 (1978-09-22)
- H. ZAHN, B. SEIDEL: "Weitere Oligoester der Terephthalsäure mit Glykol" MAKROMOLEKULARE CHEMIE, Bd. 29, 1959, Seiten 70-92, XP002578584
- Broschüre "PolyTHF: Supporting your growth", BASF

## Beschreibung

Die Erfindung betrifft die Verwendung von Mischungen enthaltend Polyole, ausgewählt aus der Gruppe 1,6-Hexandiol und Trimethylolpropan oder Mischungen davon, in einem Verfahren zur Herstellung von Polyestern.

Polyole, wie insbesondere aliphatische Di-, Tri- oder Tetrahydroxyverbindungen, besitzen aufgrund ihrer vielfältigen Verwendungsmöglichkeiten in der Technik hohe Bedeutung. Sie gelten als unter Nonnalbedingungen oxidationsbeständig und werden daher typischerweise unter Luft gelagert.

Werden solche Polyalkohole nun für Folgereaktionen eingesetzt, bei denen, z.B. mit dem Ziel der Esterbildung, saure Bedingungen geschaffen werden, so kommt es häufig trotz typischerweise hoher Reinheit des eingesetzten Polyols zu Verfärbungen, die das Folgeprodukt unbrauchbar oder unverkäuflich machen. EP0572256A2 offenbart ein Verfahren zur Polyesterherstellung, wobei phosphororganische Verbindungen und ein polyfunktionales Isocyanat zur Vermeidung von Verfärbungen zugegeben werden.

Es bestand daher das Bedürfnis, ein Verfahren bereitzustellen, das solche unerwünschten Verfärbungen vermindert.

Es wurde nun gefunden, dass durch die Verwendung von Mischungen enthaltend Polyole, ausgewählt aus der Gruppe 1,6-Hexandiol und Trimethylolpropan oder Mischungen davon, und zumindest eine oder mehrere Verbindungen der Formel (I) oder (II) 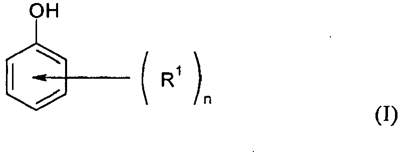 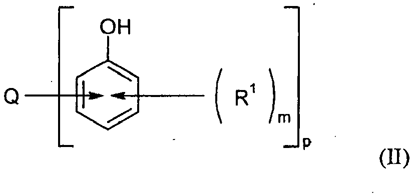 in denen
- n: für 1, 2, 3 oder 4, bevorzugt für 2 oder 3 steht
- m: für 1, 2 oder 3, bevorzugt für 1 oder 2 steht
- p: für 2 steht
- Q: für Schwefel oder C₁-C₄-Alkylen, bevorzugt für C₁-C₄-Alkylen steht und
- R¹: jeweils unabhängig von gegebenenfalls vorhandenen anderen Resten R¹ für C₁-C₈-Alkyl oder C₁-C₈-Alkoxy steht,
wobei weiterhin zumindest eine ortho-Position der beiden Phenylringe gemäß Formel (II) bzw. des Phenylrings gemäß Formel (I) mit einem sekundären oder tertiären Rest substituiert sein muss in einem. Verfahren zur Herstellung von Polyestern die unerwünschten Verfärbungen vermindert werden können.

Alkyl beziehungsweise Alkylen beziehungsweise Alkoxy bedeutet jeweils unabhängig einen geradkettigen, verzweigten oder unverzweigten, gegebenenfalls cyclischen, Alkyl- beziehungsweise Alkylen- beziehungsweise Alkoxy-Rest.

C₁-C₈-Alkyl steht beispielsweise für Methyl, Ethyl, n-Propyl, iso-.Propyl, n-Butyl, sec.-Butyl und tert.-Butyl, n-Pentyl, 1-Methylbutyl, 2-Methylbutyl, 3-Methylbutyl, neo-Pentyl, 1-Ethylpropyl, cyclo-Hexyl oder 2-Methylcyclohexyl, bevorzugt für Methyl, tert.-Butyl oder cyclo-Hexyl.

C₁-C₈-Alkoxy steht beispielsweise für Methoxy, Ethoxy, n-Propoxy, iso-Propoxy, n-Butoxy, sec.-Butoxy und tert.-Butoxy, neo-Pentoxy, cyclo-Hexoxy, cyclo-Pentoxy und n-Hexoxy.

C₁-C₄-Alkylen steht beispielsweise für Methylen, Ethandiyl, 1,2-Propandiyl, 1,3-Propandiyl, 1,4-Butandiyl, 2,3-Butandiyl oder 1,3-Butandiyl, bevorzugt für Methylen.

Bevorzugte Verbindungen der Formel (I) sind: 2,6-Bis[tert.-butyl]-4-methyl-phenol] (BHT), 2,4,6-Tri-t-butylphenol; 2- und 3-tert.-Butylhydroxyanisol bzw. Mischungen davon (BHA).

Bevorzugte Verbindungen der Formel (II) sind:
2,2'-Methylen-bis[4-methyl-6-(α-methylcyclohexyl)-phenol], 2,2'-Methylen-bis[4-methyl-6-cyclo-hexyl)-phenol] (ZKF), 2,2'-Methylen-bis-[6-tert.-butyl-4-ethylphenol], 2,2'-Methylen-bis[6-tert.-butyl-4-methylphenol] (BKF), 4,4'-Methylen-bis[2,6-di-tert.-butylphenol], 4,4'-Methylen-bis[6-tert.-butyl-2-methylphenol], wobei 2,2'-Methylen-bis[4-methyl-6-cyclohexyl)-phenol] und 2,2'-Methylen-bis[6-tert.-butyl-4-methylphenol] noch weiter bevorzugt sind.

Vorgenannte Verbindungen der Formeln (I) oder (II) können einzeln oder als Mischungen eingesetzt werden.

Unter dem Begriff Polyole werden im Rahmen der Erfindung Verbindungen verstanden, die ausgewählt sind aus der Gruppe Hexan-1,6-diol und Trimethylolpropan oder Mischungen davon.

Die Gesamtmenge an zugesetzten Verbindungen der Formeln (I) oder (II) beträgt beispielsweise 0,0001 bis 60 Gew.-%, bevorzugt 0,0001 bis 2,0 Gew.-%, besonders bevorzugt 0,001 bis 1,0 Gew.-%, ganz besonders bevorzugt 0,001 bis 0,1 Gew.-% und noch weiter bevorzugt 0,002 bis 0,8 Gew.-%.

Bevorzugt ist die Verwendung von Mischungen enthaltend Polyole und zumindest eine Verbindung der Formeln (I) oder (II) umfasst, wobei der Gewichtsanteil der Polyole mindestens 40 %, vorzugsweise mindestens 90 %, besonders bevorzugt mindestens 95% und ganz besonders bevorzugt mindestens 98 % entspricht und der Gesamtgehalt an Verbindungen der Formeln (I) oder (II) in der Mischung 0,0001 bis 60 Gew.-%, bevorzugt 0,0001 bis 2,0 Gew.-%, besonders bevorzugt 0,001 bis 1,0 Gew.-%, ganz besonders bevorzugt 0,001 bis 0,1 Gew.-% und noch weiter bevorzugt 0,002 bis 0,8 Gew.-% beträgt.

Mischungen, mit hohem Gehalt an Verbindung der Formeln (I) oder (II) können auch als Masterbatch eingesetzt werden, um die oben in Vorzugsbereichen genannten Endkonzentrationen leichter zu erreichen, sind ebenfalls bevorzugt als Rohstoff für die verwendlmgfähigen Mischungen.

Die eingesetzten Mischungen können fest oder flüssig sein.

Vorzugsweise enthalten die Mischungen 2 Gew.-% oder weniger, bevorzugt 1 Gew-% oder weniger an Stoffen, die keine Polyole oder Verbindungen der Formeln (I) oder (II) sind.

Die in einem Verfahren zur Herstellung von Polyestern eingesetzten Polyole eignen sich insbesondere für solche Verfahren, die unter sauren Bedingungen durchgeführt werden, wobei unter dem Begriff "sauer" ein pH-Wert verstanden wird, der bezogen auf eine wässrige Vergleichsskala unter Standardbedingungen unter 7,0 liegt. Die vorgenannten Vorzugsbereiche für die Gehalte an Polyolen und Verbindungen der Formeln (I) und (II) gelten dabei in gleicher Weise.

Solche Verfahren zur Herstellung von Polyestern sind dem Fachmann hinlänglich bekannt und umfassen z.B. die Herstellung von Polyestern durch Umsetzung von Polyolen mit Polycarbonsäuren unter Wasserabspaltung gegebenenfalls in Gegenwart von Katalysatoren. Dabei können die Reaktionstemperaturen beispielsweise 20° bis 240°C, vorzugsweise 40 bis 190°C betragen.

Überaschenderweise haben die zugesetzten Verbindungen der Formeln (I) und (II) keinen negativen Einfluss auf den Veresterungsprozess selbst, vermeiden jedoch selbst bei hohen Prozesstemperaturen weitestgehend unerwünschte Verfärbungen. Erfindungsgemäß ist es dabei im Regelfall möglich, die Verfärbung auf eine Farbzahl von weniger als 100 APHA, bevorzugt auf 60 oder weniger zu beschränken.

Die Erfindung wird anhand der Beispiele näher erläutert ohne sie jedoch darauf zu beschränken.

### Versuche:

Jeweils 100 g der zu untersuchenden Mischung aus 1,6-Hexandiol und Stabilisator wurden in einen 1 1 Vierhalskolben vorgelegt und mittels eines Heizpilzes unter Rühren auf 130°C für 4 h erhitzt. Anschließend wurden unter ständiger Stickstoffüberleitung 100 g Adipinsäure in die entstandene Schmelze gegeben, wobei sich diese auf ca. 80 bis 90°C abkühlte. Anschließend wurde innerhalb von 5 Minuten auf 175°C erhitzt und 10 Minuten bei dieser Temperatur reagieren gelassen. Der bei der Veresterung entstehende Wasserdampf wurde abgeleitet. Nach Ende der Reaktionszeit wurde die Farbzahl der Schmelze bei 130°C in einer 11 mm Rundküvette bestimmt. Die Kalibrierung erfolgte jeweils gegen Wasser (APHA-Farbzahl 0) jeweils unmittelbar vor der Messung.

Die Versuchsergebnisse sind in nachstehender Tabelle zusammengefasst:

| **Beispiel** | **Menge** | **Stabilisator** | **APHA Farbzahl** |
|---|---|---|---|
| 1 | 0 | keiner | 700 |
| 2** | 0,05 % | Cyclohexen-3-ylidenmethyl-benzylether | 500 |
| 3** | 0,05 % | Pentaerythrol-bis[cyclohexen-3-yl-1-formyl]acetal | 100 |
| 4 | 1 % | 2,6-Bis[tert.-butyl]-4-methyl-phenol] | 15 |
| 5* | 0,1 % | 2,6-Bis[tert.-butyl]-4-methyl-phenol]] | 18 |
| 6 | 0,05 % | 2,6-Bis[tert.-butyl]-4-methyl-phenol] | 9 |
| 7 | 0,025 % | 2,6-Bis[tert.-butyl]-4-methyl-phenol] | 23 |
| 8 | 0,01 % | 2,6-Bis[tert.-butyl]-4-methyl-phenol] | 56 |
| 9 | 0,025 % | 2,2'-Methylen-bis[6-tert.-butyl-4-methylphenol] | 50 |
| 10 | 0,025 % | 2,2'-Methylen-bis[4-methyl-6-cyclohexyl)-phenol] | 60 |
| 11** | 0,025 % | Mischung von alkylierten Styrylphenolen | 500 |
| 12** | 0,025 % | Mischung von Styrylphenolen | 700 |
| 13 | 0,025 % | Mischung aus 2- und 3-tert.-Butylhydroxyanisol | 60 |

| | | | |
|---|---|---|---|
| * Einsatz von Capronsäure statt Adipinsäure ** Beispiele zum Vergleich | | | |

Eingesetzte Antioxidantien für nicht erfindungsgemäße Beispiele:

| | |
|---|---|
| Cyclohexen-3-ylidenmethyl-benzylether | Pentaerythrol-bis[cyclohexen-3-yl-1-formyl]acetal |
| 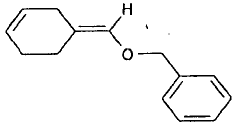 | 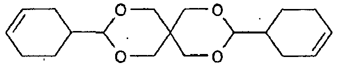 |
| Mischung von Styrylphenolen | Mischung von alkylierten Styrylphenolen |
| 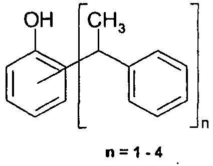 | 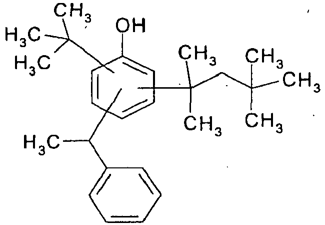 |

## Patentansprüche

1. Verwendung von Mischungen enthaltend Polyole, ausgewählt aus der Gruppe 1,6-Hexandiol, Trimethylolpropan oder Mischungen davon,
und zumindest eine oder mehrere Verbindungen der Formel (I) oder (II) 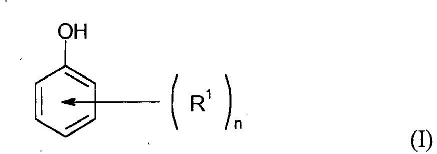 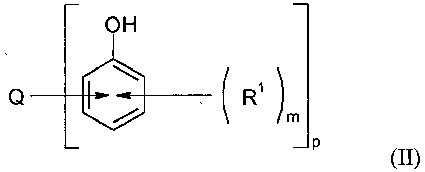 in denen
n für 1, 2, 3 oder 4 steht
m für 1, 2 oder 3 steht
p für 2 steht
Q für Schwefel oder Methylen steht und
R¹ jeweils unabhängig von gegebenenfalls vorhandenen anderen Resten R¹ für C₁-C₈-Alkyl oder C₁-C₈-Alkoxy steht,
wobei weiterhin zumindest eine ortho-Position der beiden Phenylringe gemäß Formel (II) bzw. des Phenylrings gemäß Formel (I) mit einem sekundären oder tertiären Rest substituiert sein muss, in einem Verfahren zur Herstellung von Polyestern.

2. Verwendung nach Anspruch 1, **dadurch gekennzeichnet, dass** als Verbindungen der Formel (I) oder (II) 2,6-Bis[tert.-butyl]-4-methyl-phenol] (BHT), 2,4,6-Tri-t-butylphenol; 2- und 3-tert.-Butylhydroxyanisol bzw. Mischungen davon (BHA), 2,2'-Methylen-bis[4-methyl-6-(α-methylcyclohexyl)-phenol] (ZKF), 2,2'-Methylen-bis[4-methyl-6-(α-methylcyclohexyl)phenol], 2,2'-Methylen-bis[6-tert.-butyl-4-ethylphenol], 2,2'-Methylen-bis[6-tert.-butyl-4-methylphenol] (BKF), 4,4'-Methylen-bis[2,6-di-tert.-butylphenol], 4,4'-Methylen-bis[6-tert.-butyl-2-methylphenol] oder Mischungen davon eingesetzt werden.

## Claims

1. Use of mixtures containing polyols selected from the group consisting of 1,6-hexanediol, trimethylolpropane and mixtures thereof plus at least one or more compounds of the formula (I) or (II) 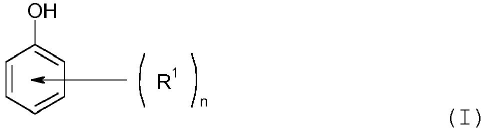 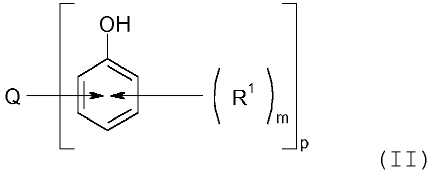 where
n is 1, 2, 3 or 4,
m is 1, 2 or 3,
p is 2,
Q is sulphur or methylene, and
the radicals R¹ are each, independently of any other radicals R¹ present, C₁-C₈-alkyl or C₁-C₈-alkoxy, where at least one ortho position of the two phenyl rings in the formula (II) or the phenyl ring in the formula (I) must be substituted by a secondary or tertiary radical, in a process for preparing polyesters.

2. Use according to Claim 1, **characterized in that** 2,6-bis[tert-butyl]-4-methylphenol] (BHT), 2,4,6-tri-t-butylphenol; 2- and 3-tert-butylhydroxyanisole and mixtures thereof (BHA), 2,2'-methylenebis[4-methyl-6-(α-methylcyclohexyl)phenol] (ZKF), 2,2'-methylenebis[4-methyl-6-(α-methylcyclo-hexyl)phenol], 2,2'-methylenebis[6-tert-butyl-4-ethylphenol], 2,2'-methylenebis[6-tert-butyl-4-methylphenol] (BKF), 4,4'-methylenebis[2,6-di-tert-butylphenol], 4,4'-methylenebis[6-tert-butyl-2-methylphenol] or mixtures thereof are used as compounds of the formula (I) or (II).

## Revendications

1. Utilisation de mélanges contenant des polyols, choisis dans le groupe constitué par le 1,6-hexanediol, le triméthylolpropane ou leurs mélanges,
et au moins un ou plusieurs composés de formule générale (I) ou (II) 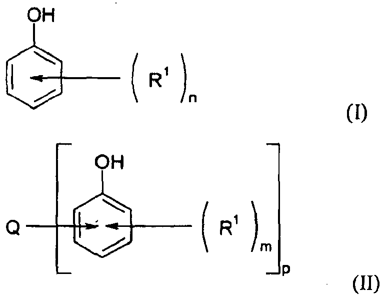 où
n vaut 1, 2, 3 ou 4
m vaut 1, 2 ou 3
p vaut 2
Q représente soufre ou méthylène et
R¹ représente, à chaque fois indépendamment des autres radicaux R¹ le cas échéant présents, C₁-C₈-alkyle ou C₁-C₈-alcoxy,
et où en outre au moins une position ortho des deux cycles phényle selon la formule (II) ou du cycle phényle selon la formule (I) doit être substituée par un radical secondaire ou tertiaire, dans un procédé pour la préparation de polyesters.

2. Utilisation selon la revendication 1, **caractérisée en ce qu'**on utilise, comme composés de formule (I) ou (II), le 2,6-bis[tert-butyl]-4-méthylphénol] (BHT), le 2,4,6-tri-t-butylphénol, le 2-tert-butylhydroxyanisol et le 3-tert-butylhydroxyanisol ou, selon le cas leurs mélanges (BHA), le 2,2'-méthylènebis[4-méthyl-6-(α-méthylcyclohexyl)-phénol] (ZKF), le 2,2'-méthylènebis[4-méthyl-6-(α-méthylcyclohexyl)-phénol], le 2,2'-méthylènebis[6-tert-butyl-4-éthylphénol], le 2,2'-méthylènebis[6-tert-butyl-4-méthylphénol] (BKF), le 4,4'-méthylènebis[2,6-di-tert-butylphénol], le 4,4'-méthylènebis[6-tert-butyl-2-méthylphénol] ou leurs mélanges.
